# EUROPEAN PATENT SPECIFICATION

(11) **EP 4 093 363 B1**
(45) Date of publication and mention of the grant of the patent: **08.07.2026**
(21) Application number: 21705364.4
(22) Date of filing: 21.01.2021
(51) Int. Cl.: A61J 1/22, A61J 1/20, A61M 5/145, A61M 5/178

(54) **SYSTEM AND DEVICE FOR DRUG PREPARATION**
SYSTEM UND VORRICHTUNG ZUR ARZNEIZUBEREITUNG
SYSTÈME ET DISPOSITIF DE PRÉPARATION DE MÉDICAMENT

(30) Priority: 22.01.2020 US 202062964297 P
(43) Date of publication of application: 30.11.2022
(73) Proprietor: Becton, Dickinson and Company, Franklin Lakes, NJ 07417 (US)
(72) Inventor: SAINATH, Paavana, Fairfield, New Jersey 07004 (US); WITT, Erik Kurt, Oakland, New Jersey 07436 (US); PONCE DE LEON, Philip, Brooklyn, New York 11217 (US); NELSON, Mark, Andrew, Harrison, New Jersey 07029 (US); BALJI, Jack, Mahwah, New Jersey 07430 (US); SANDMANN, Christian, Wayne, New Jersey 07470 (US); OSHINSKI, Matthew, Oak Ridge, New Jersey 07438 (US); PUPLAMPU, Adinor, Emerson, New Jersey 07630 (US)
(74) Representative: Germain Maureau
(86) International application number: PCT/US2021/014311
(87) International publication number: WO 2021/150677

(56) References cited:
- EP-A1- 3 284 492
- EP-A2- 2 351 590
- WO-A1-2017/156523
- WO-A1-2019/136163
- US-A1- 2003 233 069
- US-A1- 2018 193 552

## Description

### CROSS-REFERENCE TO RELATED APPLICATION

The present application claims priority to United States Provisional Application Serial No. 62/964,297, entitled "System and Device for Drug Preparation", filed January 22, 2020.

### BACKGROUND OF THE INVENTION

### Field of the Disclosure

The present application relates generally to a system and device for drug preparation.

### Description of the Related Art

Patient specific medication is prepared by pharmacy technicians in a sterile field based on a printed preparation label that is approved by a pharmacist. The process of preparation is largely manual, although there are various automation technologies on the market. These automation technologies generally fall into three categories: workflow software; compounders; and fully-automated robots. Workflow software provides order management, prioritization, approvals within the pharmacy, and documentation. Workflow software solutions typically require some level of IT integration as well as hardware, such as a scale, a camera, and monitors. Compounder solutions are semi-automated solutions that automate certain key aspects of the sterile compounding process with significant technician intervention. Fully-automated robotic solutions are complex and typically require significant reconfiguration of the central pharmacy, including construction.

US2003/233069 discloses to an infusion pump having a housing that supports a syringe assembly, a user interface, a power supply, a drive mechanism, and a syringe sensor system.

EP3 284 492 discloses a contrast medium and/or saline injector having an injection head.

EP2 351 590 discloses a computer controlled electric syringe having an injection needle (with a needle fixing member) attached thereto, and includes a cartridge holder (for holding a cartridge therein), a cartridge holder socket, an operation indicator, a main case, a start switch, an aspiration switch, an information display, a mode/speed setting switch, a cartridge/melody setting switch, and a power switch.

WO2017/156523 discloses an injection assembly that includes an injector and a dose module inserted into the injector.

WO2019/136163 discloses a handheld therapeutic agent delivery device having an actuator component and a therapeutic agent delivery system operably engaged in a receiving space of the actuator component.

US 2018/193552 discloses a drug transfer device.

### SUMMARY OF THE INVENTION

In accordance with one aspect of the invention, a drug transfer device includes a syringe interface configured to engage with one end of a syringe barrel, and a drive member movable relative to the syringe interface, with the drive member configured to move a stopper within a syringe barrel when a syringe barrel is engaged with the syringe interface. The drug transfer device further includes a motor configured to move the drive member, a position sensor configured to measure a position of the drive member, a wireless communication device configured to send and receive information, an information reader configured to receive information from an information target, and at least one processor in communication with the motor, the position sensor, the wireless communication device, and the information reader.

In certain configurations, the drug transfer device also includes a rechargeable battery. Optionally, the information reader of the drug transfer device includes at least one of a barcode reader, an RFID reader, and an NFC reader. In other configurations, the wireless communication device is configured to communicate via at least one of Bluetooth, Wi-Fi, and NFC. In certain configurations, when the motor moves the drive member, it moves the stopper within the syringe barrel to either aspirate fluid into the syringe or expel fluid from the syringe barrel. The position sensor measures the position of the plunger interface of the drive member such that the position of the syringe barrel is determined. In certain configurations, the processor is adapted to communicate with the motor to move the drive member to a position that corresponds to a position based on the position sensor which corresponds to information from the wireless communication device.

In accordance with another aspect, a system for drug preparation includes a drug transfer device including a syringe interface configured to receive a plurality of syringe barrel sizes. The system also includes a drive member movable relative to the syringe interface, the drive member is configured to move a stopper within a syringe barrel when a syringe barrel is engaged with the syringe interface. The system also includes a motor configured to move the drive member, a position sensor configured to measure a position of the drive member, a wireless communication device configured to send and receive information, and an information reader. The system also includes at least one processor in communication with the motor, the position sensor, the wireless communication device, and the information reader. The system further includes a medication workflow system in communication with the drug transfer device via the wireless communication device, and at least one information target configured to be read by the information reader of the drug transfer device.

In certain configurations, the information target comprises a barcode. Optionally, the system may further include a printer in communication with the drug transfer device and/or the medication workflow system. In still further configurations, the information reader may include at least one of a barcode reader, an RFID reader, and an NFC reader.

In other configurations, the wireless communication device is configured to communicate via at least one of Bluetooth, WiFi, and NFC. In accordance with certain aspects of the present invention, when the motor moves the drive member, it moves the stopper within the syringe barrel to either aspirate fluid into the syringe or expel fluid from the syringe barrel. The position sensor may measure the position of the plunger interface of the drive member such that the position of the syringe barrel is determined. The processor may be adapted to communicate with the motor to move the drive member to a position that corresponds to a position based on the position sensor which corresponds to information from the wireless communication device.

### BRIEF DESCRIPTION OF THE DRAWINGS

The above-mentioned and other features and advantages of this disclosure, and the manner of attaining them, will become more apparent and the disclosure itself will be better understood by reference to the following descriptions of aspects of the disclosure taken in conjunction with the accompanying drawings, wherein:
FIG. 1 is a schematic view of a drug transfer device according to one aspect of the present application.
FIG. 2 is a schematic view of a system for drug preparation.

Corresponding reference characters indicate corresponding parts throughout the several views. The exemplifications set out herein illustrate exemplary aspects of the disclosure, and such exemplifications are not to be construed as limiting the scope of the disclosure in any manner.

### DETAILED DESCRIPTION

The following description is provided to enable those skilled in the art to make and use the described aspects contemplated for carrying out the invention. Various modifications, equivalents, variations, and alternatives, however, will remain readily apparent to those skilled in the art.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary aspects of the invention. Hence, specific dimensions and other physical characteristics related to the aspects disclosed herein are not to be considered as limiting. All numbers and ranges used in the specification and claims are to be understood as being modified in all instances by the term "about". By "about" is meant plus or minus twenty-five percent of the stated value, such as plus or minus ten percent of the stated value. However, this should not be considered as limiting to any analysis of the values under the doctrine of equivalents.

Unless otherwise indicated, all ranges or ratios disclosed herein are to be understood to encompass the beginning and ending values and any and all subranges or subratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less. The ranges and/or ratios disclosed herein represent the average values over the specified range and/or ratio.

The terms "first", "second", and the like are not intended to refer to any particular order or chronology, but refer to different conditions, properties, or elements.

As used herein, "at least one of" is synonymous with "one or more of". For example, the phrase "at least one of A, B, and C" means any one of A, B, or C, or any combination of any two or more of A, B, or C. For example, "at least one of A, B, and C" includes one or more of A alone; or one or more B alone; or one or more of C alone; or one or more of A and one or more of B; or one or more of A and one or more of C; or one or more of B and one or more of C; or one or more of all of A, B, and C.

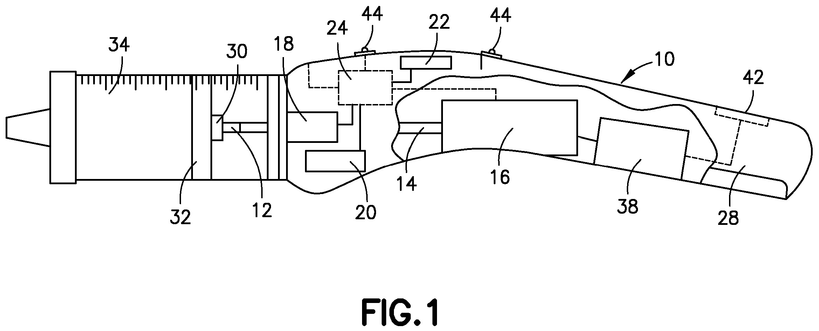

Referring to FIG. 1, a drug transfer device 10 according to one aspect or embodiment of the present application includes a syringe interface 12, a drive member 14, a motor 16, a position sensor 18, a wireless communication device 20, an information reader 22, and a processor 24. The drug transfer device 10 is configured to receive a plurality of syringe barrel sizes. As discussed in more detail below, the drug transfer device 10 is utilized for transferring and dosing medication between various containers, such as transferring a dose from a vial to a syringe to an intravenous bag or container. The drug transfer device 10 may be utilized in connection with other components such a vial adapter, syringe adapter, patient connector, etc. The motor 16, the position sensor 18, the wireless communication device 20, the information reader 22, and the processor 24 are positioned within a housing 28, although these components or portions of these components may be positioned outside of the housing 28. A portion of the drive member 14 is also received within the housing 28, although other suitable arrangements may be utilized.

The drive member 14 is movable relative to the syringe interface 12. The drive member 14 is movable such that a plunger interface 30 of the drive member 14 can be moved toward the housing 28 or away from the housing 28. The drive member 14 is configured to move a stopper 32 within a syringe barrel 34 when the syringe barrel 34 is engaged with the syringe interface 12, as shown in FIG. 1. The motor 16 is configured to move the drive member 14, which, in turn, moves the stopper 32 within the syringe barrel 34 to either aspirate fluid into the syringe barrel 34 or expel fluid from the syringe barrel 34. The position sensor 18 is configured to measure a position of the drive member 14. In particular, the position sensor 18 measures the position of the plunger interface 30 of the drive member 14 such that the position of the stopper 32 of the syringe barrel 34 can be precisely determined. The wireless communication device 20 is configured to send and receive information. The information may be medication dosage information, dosage documentation, patient documentation, a status of the drug transfer device, etc. The information reader 22 is configured to receive information from an information target 36. The processor 24 is in communication with the motor 16, the position sensor 18, the wireless communication device 20, and the information reader 22. The processor 24 may be one or more processors. The processor 24 may be a microcontroller, although other suitable arrangements may be utilized. The drug transfer device 10 includes a rechargeable battery 38, although other suitable batteries, external power supplies, and power sources may be used in addition to or alternatively to the rechargeable battery 38.

The information reader 22 may be at least one of a barcode reader, an RFID reader, and an NFC reader, although other suitable information readers may be utilized. The information target 36 may be at least one of a barcode, an RFID tag, and an NFC tag, although other suitable information targets may be utilized. The wireless communication device 20 is configured to communicate via at least one of Bluetooth, Wi-Fi, and NFC. The information target 36 is scanned by the information reader 22. For example, a medication label 40 can be provided with the information target 36, which is read by the information reader 22 of the drug transfer device 10. The drive member 14 of the drug transfer device 10 can then be manually, semi-manually, or automatically actuated to aspirate a dose of medication into the syringe barrel 34 based on the information target 36 of the medication label 40. The required dose of medication may be determined based on stored information on the drug transfer device 10 or may be accessed from a server, or other suitable device remotely, via the wireless communication device 20.

Referring again to FIG. 1, the drug transfer device 10 may further include a display 42 and user input interface 44, such as a touch screen, button, knob, etc. The display 42 and the user input interface 44 are connected to the processor 24, with the display 42 configured to display information received by the drug transfer device 10 or stored on the drug transfer device 10. The display 42 and the user input interface 44 may be utilized to operate the drug transfer device 10, although other suitable arrangements, such as remote control of the drug transfer device 10 via a computer terminal or mobile device, may be utilized to operate the drug transfer device 10.

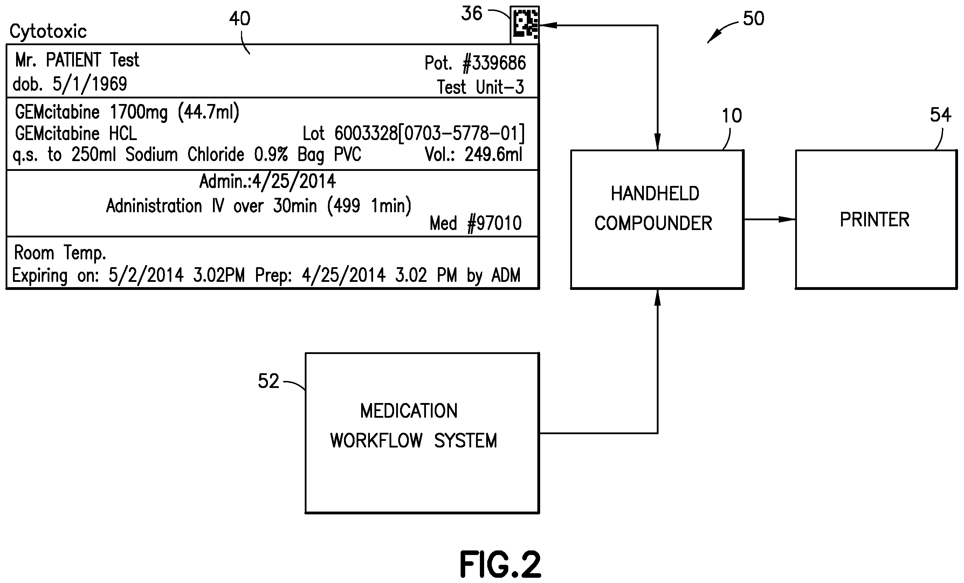

Referring to FIGS. 1 and 2, a system 50 for drug preparation according to one aspect or embodiment of the present application includes the drug transfer device 10 of FIG. 1, a medication workflow system 52 in communication with the drug transfer device 10 via the wireless communication device 20, and at least one information target 36 configured to be read by the information reader 22 of the drug transfer device 10. The medication workflow system 52 may be the BD Cato^{®} system available from Becton, Dickinson and Company, although other suitable medication workflow systems may be utilized. The medication workflow system 52 may be configured to enable order management, prioritization, approvals within the pharmacy, documentation, and other suitable functions.

The system 50 may further include a printer 54 in communication with the drug transfer device 10 and/or the medication workflow system 52. The printer 54 may be wirelessly and/or physically connected to the drug transfer device 10 and/or medication workflow system 52.

The system 50 is configured to enable compounding patient specific doses of medication while ensuring a prescribed medication and medication dose. As discussed above, the medication label 40 can be provided with the information target 36, which is read by the information reader 22 of the drug transfer device 10. The medication label 40 may be generated by the medication workflow system 52 and printed via the printer 54. After the information target 36 is read by the information reader 22, the drive member 14 of the drug transfer device 10 may be manually, semi-manually, or automatically actuated to aspirate a dose of medication into the syringe barrel 34 based on the information target 36 of the medication label 40. The required dose of medication may be determined based on stored information on the drug transfer device 10 or may be accessed from a server and/or the medication workflow system 52 via the wireless communication device 20. The medication label 40 may be printed via the printer based on information provided by the medication workflow system 52 and/or the drug transfer device 10. The medication label 40 may be affixed to the syringe barrel 34 after compounding or a separate container. The drug transfer device 10 may communicate with the medication workflow system 52 to provide automatic documentation of the compounding process.

While this disclosure has been described as having exemplary designs, the present disclosure can be further modified within the scope of this disclosure. This application is therefore intended to cover any variations, uses, or adaptations of the disclosure using its general principles. To the extent possible, one or more features of any embodiment or aspect can be combined with one or more features of any other embodiment or aspect. Further, this application is intended to cover such departures from the present disclosure as come within known or customary practice in the art to which this disclosure pertains and which fall within the limits of the appended claims.

## Claims

1. A drug transfer device (10) comprising:
a housing (28) configured to be handheld by a user;
a syringe interface (12) configured to engage with one end of a syringe barrel (34);
a drive member (14) movable relative to the syringe interface (12), the drive member (14) configured to move a stopper (32) within the syringe barrel (34) when the syringe barrel (34) is engaged with the syringe interface (12);
a motor (16) configured to move the drive member (14);
a position sensor (18) configured to measure a position of the drive member (14);
a wireless communication device (20) configured to send and receive information;
an information reader (22) configured to receive information from an information target (36), the information target (36) readable by the information reader (22); and
at least one processor (24) in communication with the motor (16), the position sensor (18), the wireless communication device (20), and the information reader (22),
**characterized in that** the drive member (14) is configured to automatically move the stopper (32) based upon the information received from the information target (36), to aspirate fluid into the syringe barrel (34).

2. The device (10) of claim 1, further comprising a rechargeable battery (38).

3. The device (10) of claim 1, wherein the information reader (22) comprises at least one of a barcode reader, an RFID reader, and an NFC reader, and wherein the information target (36) comprises at least one of a barcode, an RFID tag, and an NFC device.

4. The device (10) of claim 1, wherein the wireless communication device (20) is configured to communicate via at least one of Bluetooth, Wi-Fi, and NFC.

5. The device (10) of claim 1, wherein when the motor (16) moves the drive member (14), it moves the stopper (32) within the syringe barrel (34).

6. The device (10) of claim 1, wherein the position sensor (18) measures the position of the plunger interface (30) of the drive member (14) such that the position of the syringe barrel (34) is determined.

7. The device (10) of claim 6, wherein the processor (24) is adapted to communicate with the motor (16) to move the drive member (14) to a position that corresponds to a position based on the position sensor (18) which corresponds to information from the wireless communication device (20).

8. A system (50) for drug preparation comprising:
the drug transfer device (10) of claim 1; and
a medication workflow system (52) in communication with the drug transfer device (10) via the wireless communication device (20), the medication workflow system (52) configured to:
store information for generating the information target (36); and
communicate with the drug transfer device (10) to document a compounding process performed by the drug transfer device (10).

9. The system (50) of claim 8, wherein the information target (36) comprises a barcode.

10. The system (50) of claim 8, further comprising a printer (54) in communication with the drug transfer device (10) and/or the medication workflow system (52), the printer configured to print out a medication label (40) that includes the information target (36) thereon.

## Patentansprüche

1. Arzneimitteltransfervorrichtung (10), umfassend:
ein Gehäuse (28), das so eingerichtet ist, dass es von einem Benutzer in der Hand gehalten werden kann;
eine Spritzenschnittstelle (12), die so eingerichtet ist, dass sie in ein Ende eines Spritzenzylinders (34) eingreift;
ein Antriebselement (14), das relativ zur Spritzenschnittstelle (12) bewegt werden kann, wobei das Antriebselement (14) so eingerichtet ist, dass es einen Anschlag (32) innerhalb des Spritzenzylinders (34) bewegt, wenn der Spritzenzylinder (34) mit der Spritzenschnittstelle (12) in Eingriff ist;
einen Motor (16), der so eingerichtet ist, dass er das Antriebselement (14) bewegt;
einen Positionssensor (18), der so eingerichtet ist, dass er eine Position des Antriebselements (14) misst;
eine drahtlose Kommunikationsvorrichtung (20), die so eingerichtet ist, dass sie Informationen sendet und empfängt;
einen Informationsleser (22), der so eingerichtet ist, dass er Informationen von einem Informationsziel (36) empfängt, wobei das Informationsziel (36) vom Informationsleser (22) gelesen werden kann; und
mindestens einen Prozessor (24) in Kommunikation mit dem Motor (16), dem Positionssensor (18), der drahtlosen Kommunikationsvorrichtung (20) und dem Informationsleser (22),
**dadurch gekennzeichnet, dass** das Antriebselement (14) so eingerichtet ist, dass es den Anschlag (32) basierend auf den vom Informationsziel (36) empfangenen Informationen automatisch bewegt, um Flüssigkeit in den Spritzenzylinder (34) anzusaugen.

2. Vorrichtung (10) nach Anspruch 1, ferner umfassend eine wiederaufladbare Batterie (38).

3. Vorrichtung (10) nach Anspruch 1, wobei der Informationsleser (22) mindestens einen von einem Barcodeleser, einem RFID-Leser und einem NFC-Leser umfasst, und wobei das Informationsziel (36) mindestens eines von einem Barcode, einem RFID-Tag und einer NFC-Vorrichtung umfasst.

4. Vorrichtung (10) nach Anspruch 1, wobei die drahtlose Kommunikationsvorrichtung (20) so eingerichtet ist, dass sie über mindestens eines von Bluetooth, WLAN und NFC kommuniziert.

5. Vorrichtung (10) nach Anspruch 1, wobei der Motor (16), wenn er das Antriebselement (14) bewegt, den Anschlag (32) innerhalb des Spritzenzylinders (34) bewegt.

6. Vorrichtung (10) nach Anspruch 1, wobei der Positionssensor (18) die Position der Kolbenschnittstelle (30) des Antriebselements (14) so misst, dass die Position des Spritzenzylinders (34) bestimmt wird.

7. Vorrichtung (10) nach Anspruch 6, wobei der Prozessor (24) so ausgelegt ist, dass er mit dem Motor (16) kommuniziert, um das Antriebselement (14) in eine Position zu bewegen, die einer Position basierend auf dem Positionssensor (18) entspricht, die Informationen von der drahtlosen Kommunikationsvorrichtung (20) entspricht.

8. System (50) zur Arzneimittelzubereitung, umfassend:
die Arzneimitteltransfervorrichtung (10) nach Anspruch 1; und
ein Medikamenten-Workflow-System (52), das über die drahtlose Kommunikationsvorrichtung (20) mit der Arzneimitteltransfervorrichtung (10) kommuniziert, wobei das Medikamenten-Workflow-System (52) für Folgendes eingerichtet ist:
Speichern von Informationen zum Erzeugen des Informationsziels (36); und
Kommunizieren mit der Arzneimitteltransfervorrichtung (10), um einen von der Arzneimitteltransfervorrichtung (10) durchgeführten Compoundierungsprozess zu dokumentieren.

9. System (50) nach Anspruch 8, wobei das Informationsziel (36) einen Barcode umfasst.

10. System (50) nach Anspruch 8, ferner umfassend einen Drucker (54) in Kommunikation mit der Arzneimitteltransfervorrichtung (10) und/oder dem Medikamenten-Workflow-System (52), wobei der Drucker so eingerichtet ist, dass er ein Medikamentenetikett (40) druckt, das das darauf befindliche Informationsziel (36) enthält.

## Revendications

1. Dispositif de transfert de médicament (10) comprenant :
un boîtier (28) configuré pour être tenu à la main par un utilisateur ;
une interface de seringue (12) configurée pour s'engager avec une extrémité d'un cylindre de seringue (34) ;
un élément d'entraînement (14) mobile par rapport à l'interface de seringue (12), l'élément d'entraînement (14) étant configuré pour déplacer un bouchon (32) à l'intérieur du cylindre de seringue (34) lorsque le cylindre de seringue (34) est engagé avec l'interface de seringue (12) ;
un moteur (16) configuré pour déplacer l'élément d'entraînement (14) ;
un capteur de position (18) configuré pour mesurer une position de l'élément d'entraînement (14) ;
un dispositif de communication sans fil (20) configuré pour envoyer et recevoir des informations ;
un lecteur d'informations (22) configuré pour recevoir des informations d'une cible d'informations (36), la cible d'informations (36) pouvant être lue par le lecteur d'informations (22) ; et
au moins un processeur (24) en communication avec le moteur (16), le capteur de position (18), le dispositif de communication sans fil (20) et le lecteur d'informations (22),
**caractérisé en ce que** l'élément d'entraînement (14) est configuré pour déplacer automatiquement le bouchon (32) sur la base des informations reçues de la cible d'informations (36), afin d'aspirer du fluide dans le cylindre de seringue (34).

2. Dispositif (10) selon la revendication 1, comprenant en outre une batterie rechargeable (38).

3. Dispositif (10) selon la revendication 1, dans lequel le lecteur d'informations (22) comprend au moins l'un parmi un lecteur de code à barres, un lecteur RFID et un lecteur NFC, et dans lequel la cible d'informations (36) comprend au moins l'un parmi un code à barres, une étiquette RFID et un dispositif NFC.

4. Dispositif (10) selon la revendication 1, dans lequel le dispositif de communication sans fil (20) est configuré pour communiquer via au moins l'un parmi Bluetooth, Wi-Fi et NFC.

5. Dispositif (10) selon la revendication 1, dans lequel, lorsque le moteur (16) déplace l'élément d'entraînement (14), il déplace le bouchon (32) à l'intérieur du cylindre de seringue (34).

6. Dispositif (10) selon la revendication 1, dans lequel le capteur de position (18) mesure la position de l'interface de piston (30) de l'élément d'entraînement (14) de sorte que la position du cylindre de seringue (34) soit déterminée.

7. Dispositif (10) selon la revendication 6, dans lequel le processeur (24) est adapté pour communiquer avec le moteur (16) afin de déplacer l'élément d'entraînement (14) vers une position qui correspond à une position basée sur le capteur de position (18) qui correspond à des informations du dispositif de communication sans fil (20).

8. Système (50) de préparation de médicament comprenant :
le dispositif de transfert de médicament (10) selon la revendication 1 ; et
un système de gestion du flux de travail médicamenteux (52) en communication avec le dispositif de transfert de médicament (10) via le dispositif de communication sans fil (20), le système de gestion du flux de travail médicamenteux (52) étant configuré pour :
stocker des informations afin de générer la cible d'informations (36) ; et
communiquer avec le dispositif de transfert de médicament (10) afin de documenter un processus de préparation effectué par le dispositif de transfert de médicament (10).

9. Système (50) selon la revendication 8, dans lequel la cible d'informations (36) comprend un code à barres.

10. Système (50) selon la revendication 8, comprenant en outre une imprimante (54) en communication avec le dispositif de transfert de médicament (10) et/ou le système de gestion du flux de travail médicamenteux (52), l'imprimante étant configurée pour imprimer une étiquette de médicament (40) qui comprend la cible d'informations (36) sur celle-ci.
